# EUROPEAN PATENT SPECIFICATION

(11) **EP 2 641 974 B1**
(45) Date of publication and mention of the grant of the patent: **13.09.2017**
(21) Application number: 12160316.1
(22) Date of filing: 20.03.2012
(51) Int. Cl.: C12Q 1/68

(54) **Signal enhancement in molecular assays**
Signalverstärkung in molekularen Assays
Amélioration du signal dans des analyses moléculaires

(43) Date of publication of application: 25.09.2013
(73) Proprietor: Technische Universität Braunschweig Carolo-Wilhelmina, 38106 Braunschweig (DE)
(72) Inventor: Tinnefeld, Philip, 80999 München (DE); Acuna, Guillermo, 38114 Braunschweig (DE)
(74) Representative: Kröncke, Rolf

(56) References cited:
- GUILLERMO P. ACUNA ET AL: "Distance Dependence of Single-Fluorophore Quenching by Gold Nanoparticles Studied on DNA Origami", ACS NANO, vol. 6, no. 4, 22 March 2012 (2012-03-22), pages 3189-3195, XP55036831, ISSN: 1936-0851, DOI: 10.1021/nn2050483
- INGO H. STEIN ET AL: "Single-Molecule Four-Color FRET Visualizes Energy-Transfer Paths on DNA Origami", JOURNAL OF THE AMERICAN CHEMICAL SOCIETY, vol. 133, no. 12, 30 March 2011 (2011-03-30), pages 4193-4195, XP55036946, ISSN: 0002-7863, DOI: 10.1021/ja1105464
- SHAWN J. TAN ET AL: "Building plasmonic nanostructures with DNA", NATURE NANOTECHNOLOGY, vol. 6, no. 5, 1 May 2011 (2011-05-01), pages 268-276, XP55036929, ISSN: 1748-3387, DOI: 10.1038/nnano.2011.49
- Jian Zhang ET AL: "Metal-Enhanced Single-Molecule Fluorescence on Silver Particle Monomer and Dimer: Coupling Effect between Metal Particles", Nano Letters, vol. 7, no. 7, 1 July 2007 (2007-07-01), pages 2101-2107, XP55114059, ISSN: 1530-6984, DOI: 10.1021/nl071084d

## Description

The present invention relates generally to signal enhancement in molecular assays. In particular, the present invention relates to an array allowing signal enhancement of single molecules. The assay is in particular a Bioassay allowing single molecule analysis of biomolecules. Preferably, the signal enhancement is a fluorescence enhancement based on an optical antenna. In another aspect, the present invention relates to a method for measuring irradiation of electromagnetic waves of single compounds. Based on the arrays and methods, it is possible to allow signal enhancement and, consequently, allow single molecule assays with high sensitivity and reproducibility.

### Background of the invention

In times of rapid medicinal progress on the one hand and exploding health care cost on the other hand, there is an enormous need for efficiency increase so that cost development does not outrun the advancement of health care. Miniaturization and parallelization are two common responses to these demands that are increasingly influencing the development of medical diagnostics and high-throughput drug-screening.

Interrogating and using single molecules represents the ultimate goal of miniaturization. Reaching the goal of single-molecule Bioassays is not merely a linear progression of common miniaturization and automation efforts for cost reduction by minimizing reagents. Single-molecule Bioassays will substantiate as new dimension of available information. This is because single-molecule assays are the only technique to provide detailed information on the properties of molecules without averaging and their dynamics can be studied without the need for synchronization. This advantage is easiest visualized considering single-molecule DNA sequencing, a technology that will be a milestone on the way to personalized medicine. If one visualized the base order a polymerase is incorporating into a synthesized DNA strand, the DNA sequence of the template would directly be obtained. If, however, two indistinguishable polymerase molecules simultaneously synthesized DNAs the sequence information would already be lost because the polymerase molecules are not synchronized.

Current techniques known as 'next-next generation sequencing', i.e. single-molecule sequencing, is in most approaches a single-enzyme or a polymerase assay and is likely becoming the first industrial single- molecule assay promoted by companies such as Helicos Biosciences or VisiGen Biotechnologies. These technologies represent only an intermediate stage towards the 'Golden Standard' since they require rinsing steps after each incorporation step. The reason for the inability to watch every incorporation step directly and add all four nucleotides simultaneously is found in the experimental methodology: it is related to the fact that the polymerase only incorporates the necessarily fluorophore-labeled nucleotides at comparatively high concentrations and these high concentrations cause a fluorescent background rendering single nucleotide identification impossible. This is not only a specific problem of single-molecule DNA sequencing but constitutes a general caveat of optical single-molecule assays. With typical sizes of observation volumes in single-molecule spectroscopy the interacting fluorophore-labeled molecules have to be at concentrations below 0.1 nM. In contrast to this demand many biomolecular interactions such as those of enzymes and substrates typically require significantly higher concentrations (µM - mM).

To extend single-molecule measurements to concentrations and affinities typical for biomolecular research, that is, in micro- to millimolar concentrations, a combination of nanofluidic structures with optics has been proposed. To confine the observation volume in three-dimensions, nano-apertures (also denoted zero-mode waveguides (ZMW)) have been introduced. These are typically sub-wavelengths diameter pinholes in thin metal films on fused silica slides. With a diameter of a few tens of nanometers no propagating light modes exist and the electric field strength is rapidly attenuated in the direction of propagation creating sub-diffraction limited observation volumes. Also nano-apertures are described in the art, commercial exploitation of nano-apertures is presently given in single-molecule sequencing by Pacific Bioscience only. In addition, apart of nano-apertures, optical antennas can be employed to enhance the electric field in a confined region (the so called hot spot) and, thus, reduce the observation volume. Optical antennas consist mostly of sub-wavelength metallic elements built with a top-down concept using typically electron beam technologies. Depending on the size, shape and arrangement of elements, optical antennas can be tuned for different wavelengths and yield an enhancement of the incoming electric field in a defined volume as described for example in Mohammadi, A. et al., New Journal of Physics 10, 105015, 2008 or by Kinkhabwala, A. et al., Nature Phot 3, 654-657, 2009.

Metal enhanced single-molecular fluorescence using silver particle monomer and dimer due to a coupling effect between metal particles is described by Zhang, J. et al. Nano Letters 2007, Vol. 7, No. 7, 2101-2107. It is described therein, that by direct coupling of oligomers with silver particles, a fluorescent dye located in between the coupled metal particles demonstrates enhancement of the fluorescence. The system described therein consists of nanoparticles coupled directly with single-stranded oligonucleotides whereby said single-stranded oligonucleotides allow hybridisation to form double stranded oligonucleotides which are coupled with the fluorescence dye. The dimer formations of the nanoparticles by hybridisation allow to generate the metal enhanced single-molecule fluorescence on silver particle dimers.

In EP 0 732 583 optochemical fluorescence sensors as well as methods for measuring the concentration of an analyte in a probe are described. Therein, metallic nanoparticles are placed on a layer whereby the labelled analytes to be measured can bind to analyte bounding molecules placed between the nanoparticles, thus, said nanoparticles allow enhancement of the fluorescence.

In addition, Aussenegg F. and Ditlbacher H., Physik Unserer Zeit, 2006, 37, 220 - 226, describe the use of metallic nanoparticles. In particular, it is described therein that plasmon may serve as auxiliary antenna for enhancing fluorescence of molecules, e.g. in the field of optical sensors. By coupling of the electromagnetically irradiated molecules with the plasmon of the nano-particles, it is possible to influence intensity and dynamic of the interaction between molecules and light. Thus, it is possible to increase the emitting rate of fluorescent molecules.

However, as identified recently by Nowotny and vanHulst, Nature Photonics, 2011, 5, 83-90, "although techniques in the field of antenna nanofabrication have progressed rapidly, the major hurdle now is how to couple antennas to active optical sources such as molecules, quantum dots and nitrogen-vacancy centres. Most work so far has relied on randomly depositing such sources onto an array of antennas, either as a monolayer or at a low 'single-molecule' concentration. Post-selection can be used to identify 'hot spots' and indeed orders of magnitude in antenna enhancement have been observed, although, the exact molecule-antenna arrangement usually remains unknown." That is, the main problem of using the above approaches is how to place individual molecules in the nano-apertures or in the hot spots of optical antennas. Particularly, in the case of nano-apertures, the metal cladding used heavily deteriorates the fluorescence signal inside the nano-apertures making reliable methods or even quantifiable distance measurement using e.g. fluorescence resonance energy transfer (FRET) apparently impossible. Many factors influence fluorescence inside nano-apertures that will strongly depend on the actual position of the fluorophore within the nano-aperture. Just to name a few important effects, the exponential attenuation of light in the nano-aperture, local enhancement of the excitation intensity due to plasmonic effects, enhancement of the emission rates and changes of the angular radiation pattern by the electromagnetic boundary conditions and cavity effects as well as fluorescence quenching due to electron and energy transfer to the metal cladding influence the brightness of the fluorophore. Thus, the possibility to carry out enzymatic or binding studies at physiological concentration has been limited. In case of sequencing, the problem described above could be minimized since only the template DNA had to be immobilized and the information which DNA sequence was immobilized in a specific nano-aperture is the result of the measurement. However, the sequencing approach is limited by Poissonian distribution so that optimally only 37 % of the nano-apertures accommodate a singe molecule. Finally, the fraction of usable holes will even be significantly smaller due to the deteriorated fluorescence in the nano-apertures.

Another critical issue about the nano-aperture approach is the need for top-down lithography with lower nm resolution. These chips have very high demands with respect to quality and reproducibility and are expensive to produce. Current approaches to use fluorescence enhancement with nano-antennas face similar problems. The nanotechnological production of the antenna structures is difficult and costly. Furthermore, the question how to place the molecules under study at the right positions in the hot spots is unresolved.

Self-assembled DNA structures, termed DNA origami technique, was first described by Rothemund et al., see WO 2005/030978. The main concept consists in providing a single DNA strand, typically of more than 5000 base pairs, termed scaffold, folded with the help of several (typically more than 100) different staple strands. The self-assembly DNA structure is formed by mixing the single DNA strand together with staple strands, thus, forming the predetermined DNA structure. The so called DNA origami technique enables nano scaffolding of the DNA to create arbitrary two and three dimensional shapes at the nanoscale. The structures are also termed wire-frame nano structures as described for example in US 2010/0216978. Typically the staple strands consist of single DNA stranding being in size of typically 20 to 50 base pairs, e.g. of around 30 base pairs. A half of each staple strand is complementary to a particular part of the scaffold, whereas the other half is also complimentary to another region of the scaffold. Thus, scaffolds and staple strands are forming the predetermined DNA structure of a desired shape.

In addition, staple strands can be modified in a way that different structures, like fluorescent dyes or an additional DNA sequence can be added. These additional DNA sequences act as specific binding sites protruding out of the DNA nanostructures. Metallic nanoparticles can be functionalized with the complimentary DNA sequence and therefore upon mixing with the DNA nanostructures these metallic nanoparticles can be bound to the DNA nanostructure at specific locations. For example, Pal et al., J. Am. Chem. Soc. 2011, 133, 17606-17609, describe the same accordingly.

It is the aim of the present invention to overcome the technical and practical problems of nano aperture and optical antenna approach to enable molecular, in particular, single-molecule studies of essentially all biomolecular interactions by signal enhancement of said molecules, for example at elevated concentrations that are relevant for typical biomolecular interactions, of compounds, like fluorophores, to enhance fluorescence detection, e.g. on single-molecule level, or to increase the time resolution of molecular assays, like biomolecular assays, or to influence positively the photostability of the compounds to be detected. That is, the method and array should allow molecular, like single-molecule analysis at concentrations of the compounds to be measured at micro- or even millimolar concentration. A first object of the present invention is to provide arrays, in particular, bioassays, allowing signal enhancement on the single-molecule level. In addition, methods are provided allowing single-molecule analysis based on signal enhancement.

### Brief description of the present invention

In a first aspect, an array is provided as defined in claim 1, said array comprising i.) a two or three dimensional structure of predetermined shape whereby the structure is formed of nucleic acid molecules in particular, DNA molecules; ii.) a) nucleic acid sequences having attached thereto metallic nanoparticles, whereby said nucleic acid sequences having attached thereto metallic nanoparticles are positioned at predetermined segments of the structured nucleic acid molecules in particular, DNA molecules, having a two or three dimensional structure of predetermined shape, and/or b) metallic nanoparticles being bound to the predetermined structure formed of nucleic acid molecules, in particular, DNA molecules, having a two or three dimensional structure of predetermined shape; whereby said metallic nanoparticles are arranged to allow formation of an area of electrical field enhancement due to plasmon resonance after excitation with electromagnetic waves; iii.) compound able to irradiate electromagnetic waves positioned in the vicinity of the metallic nanoparticles, like in between two metallic nanoparticles, which being attached to the nucleic acid sequences and/or bound to the predetermined structure, whereby said metallic nanoparticle(s) are arranged to allow formation of plasmon resonance and said compound is positioned in said area of electrical field enhancement due to plasmon resonance.

In particular, the present invention provides a bioassay allowing molecular, like single-molecule analysis of biomolecules based on a combination of self-assembling nucleic acid structures, e.g. based on DNA origami technique, and optical antenna attached to said structure. By applying this technique, it is possible to couple the signal of the irradiating compound with the enhancement ability of the optical antenna purposefully.

In another aspect, the present invention relates to an assay system or kit allowing measurement of molecular, like single-molecule fluorescence as well as the system for allowing measurement of irradiation of electromagnetic waves by the compound.

Moreover, the present invention relates to a method for the measurement of the irradiation of electromagnetic waves of a compound as defined in claim 14, the method comprising
a) providing a two or three dimensional structure of predetermined shape whereby the structure is formed by nucleic acid molecules, in particular, DNA molecules;
b1) providing nucleic acid sequences complementary to predetermined segments of the structure of a), whereby said free nucleic acid sequences have attached thereto metallic nanoparticles and, preferably, whereby at least two nucleic acid sequences having attached thereto metallic nanoparticles are provided; or
b2) providing metallic nanoparticles being bound to the two or three dimensional structure of predetermined shape whereby the structure is formed of nucleic acid molecules, in particular, DNA molecules;
c) providing a compound able to irradiate electromagnetic waves;
d) exciting the compound able to irradiate electromagnetic waves and the metallic nanoparticles with suitable electromagnetic waves;
e) measuring the irradiated electromagnetic waves of the compound;
whereby said nucleic acid sequences having attached thereto metallic nanoparticles or the metallic nanoparticles bound to the structure are arranged on the structure formed by nucleic acid molecules, in particular, DNA molecules, having a two or three dimensional structure of predetermined shape, to allow formation of an area of electrical field enhancement due to plasmon resonance after excitation with electromagnetic waves, and the compound able to irradiate electromagnetic waves is positioned in the vicinity, like in between said area of electrical field enhancement due to plasmon resonance of the metallic nanoparticles, preferably the at least two metallic nanoparticles.

To conclude, it is possible to provide an array system having predefined anchoring points for nanoparticles building the optical antenna and providing anchoring points for molecules, allowing compounds to irradiate electromagnetic waves in the hotspot of the antenna.

### Brief description of the drawings

Figure. 1 Sketch of the 3D DNA nanostructure with two gold nanoparticles of 40 nm diameter. At the equator plane a dye is placed at the line connecting the centre of both particles
Figure 2. Fluorescence image and three representative transients of spots indicated in the image. Spot a is a simple dye, b shows emission from a dye close to a single 100 nm nanoparticle, and c shows ∼90fold enhanced emission from a dye in the center of two gold nanoparticles.
Figure 3. Example of a high intensity transient in the center of two 100 nm gold nanoparticles. Blinking is induced using a reducing and oxidizing system. Off-states represent radical anions. The binning with different time resolution indicates how the high signal allows revealing intensity fluctuation down to below one microsecond. The average count rate of the ON state is 4 µs. Due to the electronic deadtime of the setup the true count rate is ∼10 MHz.
Figure 4: Correlation of intensity and lifetime for DNA origami with zero, one and two particles. All three populations can be clearly distinguished and fluorescence enhancement for the populations with nanoparticles is evident.

### Detailed description of the present invention

In a first aspect, an array is provided comprising i.) a two or three dimensional structure of predetermined shape whereby the structure is formed of nucleic acid molecules, in particular, DNA molecules; ii.) a) nucleic acid sequences having attached thereto metallic nanoparticles, whereby said nucleic acid sequences having attached thereto metallic nanoparticles are positioned at predetermined segments of the structured nucleic acid molecules in particular, DNA molecules, having a two or three dimensional structure of predetermined shape, and/or b) metallic nanoparticles being bound to the predetermined structure formed of nucleic acid molecules, in particular, DNA molecules, having a two or three dimensional structure of predetermined shape; whereby preferably at least two metallic nanoparticles are arranged to allow formation of an area of electrical field enhancement due to plasmon resonance after excitation with electromagnetic waves; iii.) compound able to irradiate electromagnetic waves positioned in the vicinity of the metallic nanoparticles, like in between two metallic nanoparticles, which being attached to the nucleic acid sequences and/or bound to the predetermined structure, whereby said preferably at least two metallic nanoparticles are arranged to allow formation of plasmon resonance and said compound is positioned in said area of electrical field enhancement due to plasmon resonance.

It has been recognized by the inventors that combining key elements from several state of the art nanotechnologies, e.g. nanophotonics, nucleic acid nanotechnology and for detecting purpose, single-molecule spectroscopy, it is possible to provide array systems for molecular, like single-molecule assays and signal enhancement and molecular, preferably single-molecule assays based on specifically positioning the compound able to irradiate electromagnetic waves in the vicinity of the metallic particles, preferably, in between the at least two metallic nanoparticles whereby said nanoparticles are arranged to allow formation of plasmon resonance.

The present invention is based on the employment of DNA nanostructures like DNA-origami, for the placement of optical antennas and single-molecules and the use of fluorescence enhancement for single-molecule biomolecular assays at elevated concentrations; in particular, elevated concentrations of the irradiating compounds, like fluorophores. Further, the present invention allows to increase the time resolution of the assays and the measured electromagnetic waves. In addition, the photostability of the compounds to be measured is increased.

The arrays and methods of the present invention allows to precisely place the irradiating compounds into the enhancing field of the optical antenna, i.e. the enhancing field, due to plasmon resonance of the metallic nanoparticles.

As used herein, the term predetermined structure formed of nucleic acid molecules identify a two or three dimensional structure of predetermined shape. Said structure is formed at least partly of nucleic acid molecules. Preferably those nucleic acid molecules are DNA-molecules. In a particular preferred embodiment, the DNA-molecules are self assembling in DNA-structures obtained by the so called DNA-origami-technique.

The term "nucleic acid molecules" includes also structures containing partly or consisting fully of other nucleic acid structures, like RNA, PNA etc. The skilled person is well aware of suitable nucleic acid molecules which can be employed for forming the self assembling DNA structure.

The array and the system as well as the method according to the present invention allow single molecule analysis due to the signal enhancement by the optical antenna. Thus, it is possible to overcome the problem of high background signals at elevated concentrations, e.g. at the micro- or millimolar concentration of the irradiating compounds when employing single molecule assays. Furthermore, an improved time resolution and and signal improvement by time gated detection is possible. Thus, the assay and methods according to the present invention allow for an improved signal to noise ratio during detection of the signal. That is, the fluorescence lifetime is shortened and, thus, one can specifically use the photons directly irradiated after the exciting pulse.

The term "electrical field enhancement due to plasmon resonance" refers to the increase of the electric field in the vicinity of a metallic nanoparticle due to the polarization of the nanoparticle induced by the incoming oscillating electric field (e.g. light).

In particular, the use of optical antenna for signal enhancement based on electrical field enhancement due to plasmon resonance is well known in the art. That is, after exciting the compound located in between the at least two metallic nanoparticles, those nanoparticles are forming a local electric field which interacts with the compound. The signal is enhanced by the local electric field formed between the metal particles allowing enhancement of the irradiated signals of the compound accordingly. Similarly, the radiative and non-radiative rates of the fluorophore are altered which can lead to a strong increase of the fluorescence quantum yield. That means that a dye that is usually a weak fluorophore becomes a good fluorophore while a good fluorophore usually stays a good fluorophore and only benefits from the excitation enhancement. Hence, new fluorophores become amenable to molecular, like single-molecule analysis. Not to be bound to theory it is submitted that due to plasmon resonance the local electric field on that metallic particles is formed. This electrical field allows enhancement of the signal irradiated from the compound in the hot spot of said particles. In this connection, the term hot spot is used herein to identify the area of electrical field enhancement.

In a preferred embodiment, the compound able to irradiate electromagnetic waves is a chromophore, in particular, a fluorophore. It is preferred, that the size of the metallic nanoparticles is below the exciting wave length to allow plasmon resonance. Preferably, the metallic nanoparticles have a mean size of from 10 nm to 500 nm preferably of from 10 nm to 200 nm.

It is preferred, that the metallic nanoparticles are in form of rods or spheres. However, it is also possible that the metallic nanoparticles may be in pyramidal form or may have an irregular shape depending on the type of signal enhancement and the method for detection of the signal.

Further, it is preferred that the metallic nanoparticles are selected from plasmonic material selected from silver, aluminium, gold, copper, palladium, and/or platinum. The skilled person is well of suitable metallic materials and suitable metallic nanoparticles allowing the formation of these in kind of optical antenna.

As used herein, the term "in the vicinity" refers to distance or area between the compound and the metallic particle being in the nanometre range, e.g. in the range of from 1 to 200 nm, like 2 to 100 or 5 to 50 nm. The skilled person is well aware of the size of the electrical field formed around the metallic nanoparticles due to Plasmon resonance. Said distance or area is also called the nearfield area.

Further, it is preferred, that the spacing of the at least two nanoparticles is from 2 nm to 50 nm, thus, signal enhancement due to electrical field enhancement based on plasmon resonance is achieved.

It is particularly preferred, that the orientation of the particles is adapted to the polarization of the exciting electromagnetic waves. The rigid structure of the predetermined structure formed of nucleic acid molecules according to the present invention, in particular, the DNA-origami structure, , having a two or three dimensional structure of predetermined shape allows to provide the necessary rigid structure for the positioning of the optical antenna and, in addition, for the positioning of the compound to be measured in the hot spot of the optical antenna.

The metallic nanoparticles may be bound to the predetermined structured nucleic acid molecules, having a two or three dimensional structure of predetermined shape by a nucleic acid sequence having attached said metallic nanoparticles. The nucleic acid sequences are able to hybridize to specific segments of the predetermined structure of nucleic acid molecules, having a two or three dimensional structure of predetermined shape. Alternatively or in addition, the metallic nanoparticles may be bound to the predetermined structure of nucleic acid molecules, in particular, DNA molecules, having a two or three dimensional structure of predetermined shape covalently, e.g. by linker groups. The metallic nanoparticles are arranged on said structure to allow formation of an area of electrical field enhancement due to plasmon resonance after exiting with electromagnetic waves as described above.

The compound able to irradiate electromagnetic waves is positioned in the vicinity of the metallic nanoparticles, like in between the two metallic nanoparticles positioned on the predetermined structure formed of nucleic acid molecules, having a two or three dimensional structure of predetermined shape. The compound is positioned in the vicinity of the metallic nanoparticles, like in between the two metallic nanoparticles in a way that the compound is in the hot spot, i.e. in the area of the electrical field enhancement due to the plasmon resonance formed by that metallic nanoparticles when excited with electromagnetic waves. That is, the compound is positioned a sufficient distance from the metallic nanoparticles to enhance the irradiated signal when excited by an irradiating source.

The compound may be a compound attached to the predetermined structure, alternatively, the compound is a compound being processed in said area of electrical field enhancement due to plasmon resonance formed by the at least two metallic nanoparticles.

In a preferred embodiment, a biochemical or chemical entity is positioned in the area of electrical field enhancement of the optical antenna. The biochemical or chemical entity may be an enzyme, a binding entity, in particular, a ligand, a receptor, an antibody, etc.. The biochemical or chemical entity is an entity which allows processing or binding of a compound able to irradiate electromagnetic waves, like a chromophore or fluorophore. For example, in case of DNA sequencing, the biochemical entity is the DNA polymerase while the compound able to irradiate electromagnetic waves, like fluorescence, is a labelled nucleotide, in particular, a labelled nucleotide labelled with a fluorophore.

In another embodiment, the biological entity may be a ligand or receptor and the compound able to irradiate electromagnetic waves may be a labelled chemical entity, e.g. a small molecule for which binding activity or binding affinity to said ligand or receptor is determined. The biochemical or chemical entity, positioned in the hot spot, preferably by a biochemical or chemical functionality present in the predetermined structure formed of nucleic acid molecules, having a two or three dimensional structure of predetermined shape, like the DNA origami. The skilled person is well aware of how to introduce respective functionalities in the desired origami positions allowing positioning of the biochemical or chemical entity in the hot spot. The functionality may be reactive group like a thiol-group, NHS-ester, amino-group, etc. Alternatively, the biochemical or chemical functionality may be a single stranded DNA allowing hybridisation at predetermined segments of the structure nucleic acid molecules. The biochemical or chemical functionality allow to position the biochemical or chemical entity in the hot spot area of the optical antenna.

Alternatively, the biochemical or chemical entity may be an antibody, or any other binding entity like a ligand or a receptor. Moreover, the chemical or biochemical entity may be a semiconductor nanoparticle or a unit for solar energy conversion.

In case of a two dimensional predetermined structure formed of nucleic acid molecules, like a two dimensional DNA origami, the metallic nanoparticles and the fluorescent dyes preferably in combination with the biochemical or chemical entity protrude into a third dimension. Thus, the hot spot allowing signal enhancement for molecular, like single molecule assays, is spaced apart from the DNA origami nanostructure and, in addition, allows to perform signal measurement at elevated concentrations of the compounds. Not to be bound to theory, the nanoparticles create a hot spot very close to the surface of the particle at the equator plane of the particle. The hot spot is formed spaced apart from the DNA origami structure for example by use of the nucleic acid sequences able to hybridize to the DNA origami and having attached the metallic nanoparticles. In addition, the biochemical or chemical entity is positioned in the hot spot of the optical antenna formed by said metallic nanoparticles with the help of nucleic acid sequences, e.g. via extended staple strands with single stranded overhangs.

In a preferred embodiment, the compound able to irradiate electromagnetic waves is not covalently bounded or directly hybridized with the metallic nanoparticles. For example in case of DNA sequencing, the labelled nucleotide is incorporated into the extending complementary nucleic acid strand by the DNA polymerase and being moved out of the hot spot when attaching the next nucleotide having a different label, like a different fluorophore.

It is preferred, that the predetermined structure formed of the nucleic acid molecules, like the DNA origami structure, having a two or three dimensional structure of predetermined shape, is attached to a surface by covalent or non covalent linkage. For example, the DNA present in the origami includes biotin molecules which may be attached to the surface with the help of its counterpart, like avidin or streptavidin. Of course, other possibilities of attaching the DNA origami to the surface can be envisaged. By attaching the predetermined structure on said surface, the structure is more rigid, thus, allowing to focus on the hot spot more precisely. That is, by attaching the predetermined structure on the surface allows to arrange the nanoparticles with respect to polarisation of the exciting light. The surface is typically a surface of a layer allowing to pass the electromagnetic waves. The skilled person is well aware over suitable layers. For example, said layers may be: glass, ITO glass, fused silica.

The array and assay according to the present invention is particularly useful in single molecule analysis based on measurement of electromagnetic waves irradiated from a compound present in the hot spot formed by the metallic nanoparticles. In a preferred embodiment, the present invention relates to a bioassay, comprising the array according to the present invention whereby said bioassay is allowing single molecule analysis of biomolecules.

The present invention further relates to an assay system comprising the array according to the present invention, a source for exciting with electronic waves the compound able to irradiate electromagnetic waves and able to induce plasmon resonance in the metallic nanoparticles, and, optionally, detection means for detecting the irradiated, preferably, the emitted electronic waves, like the fluorescence emitted by the fluorophore. The skilled person is well aware of suitable sources for exciting with electromagnetic waves, like laser or other sources. The laser may emit electromagnetic waves with specific wave length or with a brought range of electromagnetic wave length.

Typically, the electromagnetic waves according to the present invention is light, typically visible light.

For example, the assay system may be a spectroscopic system or may be a microscope. The array may also be useful in Raman spectroscopy or other types of spectroscopy, like surface enhanced Raman spectroscopy, surface enhanced resonance Raman spectroscopy, and fluorescence correlation spectroscopy. In addition, the assay system may be an automated DNA sequencer.

In addition, the present invention relates to a kit for measuring single molecule fluorescence, the kit comprising an array according to the present invention.

In another aspect, the present relates to a method for the measurement of irradiation of electromagnetic waves by compound, the method comprises
a) providing a two or three dimensional structure of predetermined shape whereby the structure is formed by nucleic acid molecules, in particular, DNA molecules;
b1) providing nucleic acid sequences complementary to predetermined segments of the structure of a), whereby said free nucleic acid sequences have attached thereto metallic nanoparticles and whereby preferably at least two nucleic acid sequences having attached thereto metallic nanoparticles are provided; or
b2) providing metallic nanoparticles being bound to the two or three dimensional structure of predetermined shape whereby the structure is formed of nucleic acid molecules, in particular, DNA molecules;
c) providing a compound able to irradiate electromagnetic waves;
d) exciting the compound able to irradiate electromagnetic waves with suitable electromagnetic waves;
e) measuring the irradiated electromagnetic waves of the compound;
whereby said nucleic acid sequences having attached thereto metallic nanoparticles or the metallic nanoparticles bound to the structure are arranged on the structure formed by nucleic acid molecules, in particular, DNA molecules, to allow formation of an area of electrical field enhancement due to plasmon resonance after excitation with electromagnetic waves, and the compound able to irradiate electromagnetic waves is positioned in said area of electrical field enhancement due to plasmon resonance of the metallic nanoparticles.

The method according to the present invention enables molecular, like single molecule measurement. In particular, the method allows to perform single molecular studies at elevated concentrations of the compounds to be tested as well as elevated concentrations of the labelled molecules. The method may be useful to study single molecules at high concentration necessary e.g. for most enzymatic and many biomolecular interaction assays. The functionality to work at biologically relevant micromolar to millimolar concentrations as enabled through the use of optical antennas that create sub-attoliter observation volumes. Further, the signal to be detected is increased. Thus, it is possible to increase time resolution. In addition, additional fluorophores become amenable to single-molecule analysis since fluorescence quantum yields can be enhanced.

The method allows to place the metallic optical antenna formed by the metallic nanoparticles, in particular, of the at least two metallic nanoparticles, and the compound able to irradiate electromagnetic waves at a precise defined position within the optical antenna, namely in the area of electrical field enhancement due to plasmon resonance of the metallic nanoparticles, thus, allowing significant signal enhancement of the compound. These precisely defined positions are based on the provision of the predetermined structure formed by the nucleic acid molecules, having a two or three dimensional structure of predetermined shape, in particular, the DNA origami structure.

The method is applicable in DNA sequencing as well as in drug development. The method as well as the array and assay system according to the present invention allows to create a platform for a single molecule lab-on-a-chip allowing parallel investigations of enzyme reactions, binding and drug screening assays in confined volumes.

The method allows to provide single molecule biomolecular assays for biochemical studies at elevated and relevant concentrations of the compounds to be analyzed. The necessary small excitation volumes will be achieved by enhancement of the electromagnetic waves, with the help of metal nanoparticles.

The particles as well as the biochemical or chemical entity for the biomolecular assay are arranged with the help of DNA nanostructures, that can be self assembled. Further binding sites are offered at specific positions, e.g. in the signal enhancement region to place said molecules to be studied.

It is preferred, that the method for the measurement of the irradiation of electronic magnetic waves of a compound allows for measurement of fluorescence, e.g. by microscope or other spectroscopic systems. In particular, the method can be applied even at micro- or millimolar concentrations of the compound irradiating the electromagnetic waves, like the fluorophore.

Thus, the drawbacks of the present assays described in the art, namely, the problem of a strong background etc. can be overcome.

The method according to the present invention is in particular a method where a biochemical or chemical entity, like an enzyme, a binding entity, in particular, a ligand, a receptor, an antibody, is positioned in the area of electrical field enhancement due to plasmon resonance of the metallic nanoparticles, preferably positioned by nucleic acid sequences hybridising to or bound to the predetermined structure formed of nucleic acid molecules, having a two or three dimensional structure of predetermined shape.

The present invention allows to provide assay systems and methods for single molecule assays wherein different geometries can be realized, a precise control of the stoichiometry, i.e. a defined number of particles and fluorephores is possible, and with a higher position control.

Typically, the fluorescent enhancement is at least of a factor of 10, e.g. at least 20, like at least of a factor of 50 times. In particular, it is possible to have a signal enhancement factor of 500 times, like 1000 times.

In the following, the present invention will be illustrated further by the way of example without a limiting the present application thereto.

### Example:

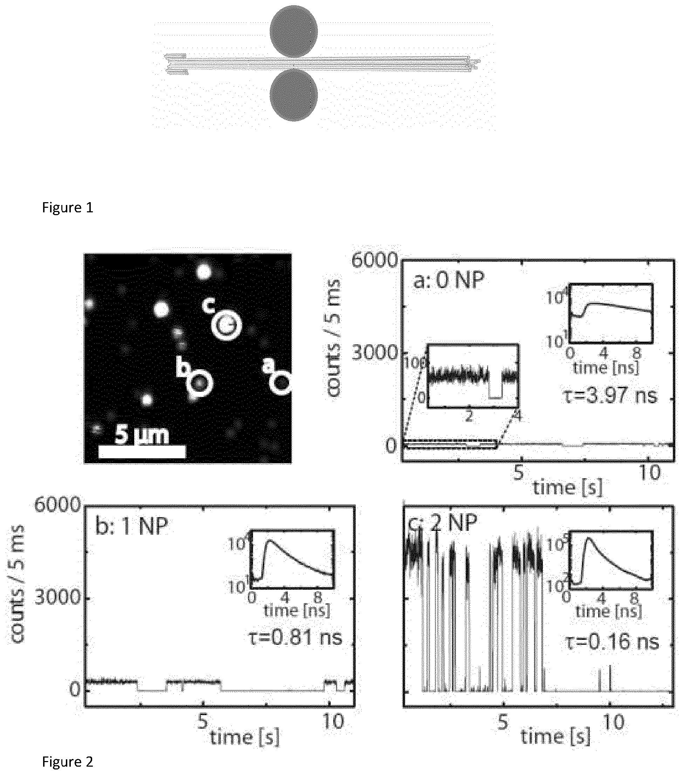

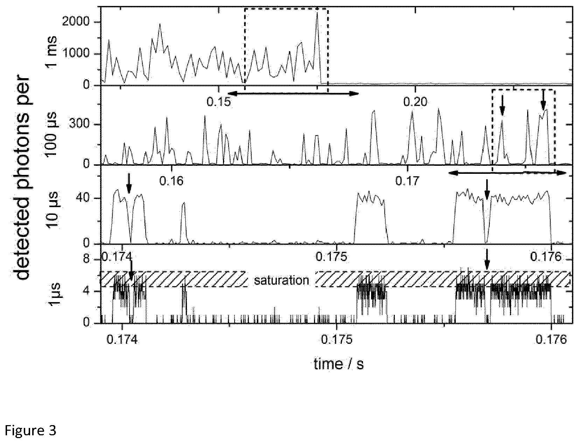

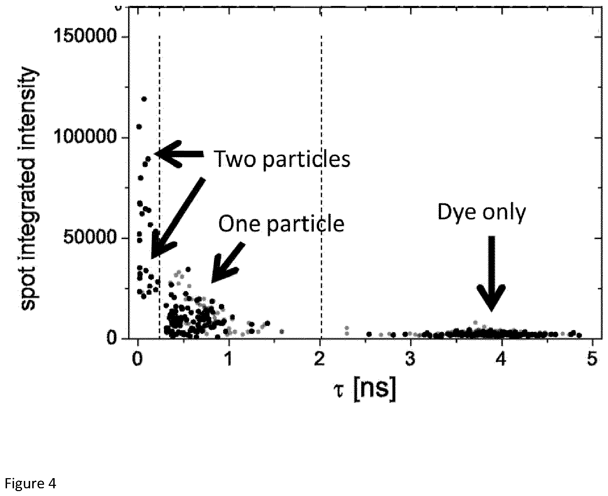

A 3D origami was constructed that allows attaching two nanoparticles of varying size on opposite sides of the DNA origami. The rocket shaped DNA origami is additionally equipped with biotins at the base for vertical placing the structure on a coverslip. One ATTO647N dye (ATTO-tec GmbH) is placed in the center between the nanoparticles see Figure 1. Single-molecule fluorescence shows spots of varying intensity on the coverslip when the rocket is partly equipped with 100 nm gold nanoparticles (Figure 2). Using fluorescence lifetime imaging, the dim spots as rockets without nanoparticles are identified. A representative fluorescence intensity transient with an average signal of about 11 kHz is shown in the right panel of figure 2. The fluorescence lifetime of 3.97 ns indicates the absence of any nanoparticle. Spot b represents a rocket with a single nanoparticle. The fluorescence intensity is increased by a factor of ∼6 and the fluorescence lifetime is significantly shortened. With two nanoparticles, the fluorescent dyes become significantly brighter with count rates of almost 1 MHz an enhancement of ∼90 fold while the fluorescent lifetime is shortened further. The increase of time resolution with higher count rates is depicted in figure 3. Figure 4 shows the correlation of fluorescence lifetime and intensity for many rockets with 0, 1, and 2 particles.

## Claims

1. An array comprising i.) a two or three dimensional structure of predetermined shape whereby the structure is formed of nucleic acid molecules in particular, DNA molecules, ; ii.) a) nucleic acid sequences having attached thereto metallic nanoparticles, whereby said nucleic acid sequences having attached thereto metallic nanoparticles are positioned at predetermined segments of the structured nucleic acid molecules in particular, DNA molecules, having a two or three dimensional structure of predetermined shape; and/or b) metallic nanoparticles being bound to the predetermined structure formed of nucleic acid molecules, in particular, DNA molecules, having a two or three dimensional structure of predetermined shape; whereby said metallic nanoparticles are arranged to allow formation of an area of electrical field enhancement due to plasmon resonance after excitation with electromagnetic waves; iii.) compound able to irradiate electromagnetic waves positioned in between two metallic nanoparticles which being attached to the nucleic acid sequences and/or bound to the predetermined structure, whereby said metallic nanoparticles are arranged to allow formation of plasmon resonance and said compound is positioned in said area of electrical field enhancement due to plasmon resonance.

2. The array according to claim 1 wherein a biochemical or chemical entity, like an enzyme, a binding entity, in particular, a ligand, a receptor, an antibody, is positioned in the area of electrical field enhancement due to plasmon resonance of the metallic nanoparticles, preferably positioned by nucleic acid sequences hybridising to or bound to the two or three dimensional structure of predetermined shape whereby the structure is formed of nucleic acid molecules.

3. The array according to any one of the preceding claims wherein the structure formed of the nucleic acid molecules, in particular, of the DNA molecules, is an origami structure, in particular, a DNA-origami structure.

4. The array according to any one of the preceding claims whereby the metallic nanoparticles are selected from plasmonic material selected from silver, aluminium, gold, copper, palladium, and/or platinum.

5. The array according to any one of the preceding claims whereby the metallic nanoparticles having a mean size of from 10 nm to 500 nm preferably of from 10 nm to 200 nm.

6. The array according to any one of the preceding claims whereby the spacing of the at least two nanoparticles attached to the nucleic acid sequences and said nucleic acid sequences are bound to the nucleic acid structure, in particular, the DNA structure, is of from 2 nm to 50 nm.

7. The array according to any one of the preceding claims whereby the compound able to irradiate electromagnetic waves is a chromophore, in particular, a fluorophore.

8. The array according to any one of the preceding claims whereby the compound able to irradiate electromagnetic waves is not covalently bonded or directly hybridised with the metallic nanoparticles.

9. The array according to anyone of the preceding claims whereby the predetermined structure formed of nucleic acid molecules, in particular, the DNA molecules, like the origami structure, is attached to a surface by covalent or non-covalent linkage.

10. The array according to any one of the preceding claims for use in single-molecule analysis.

11. A bioassay comprising an array according to any one of the preceding claims whereby said biosassay is an assay allowing single-molecule analysis of biomolecules.

12. Assay system comprising an array according to any one of claims 1 to 11, a source for exciting with electromagnetic waves the compound able to irradiate electromagnetic waves and, optionally detection means for detecting the irradiated electromagnetic waves.

13. Kit for measurement single-molecule fluorescence, the kit comprising an array according to any one of claims 1 to 11.

14. A method for the measurement of the irradiation of electromagnetic waves of a compound, the method comprising
a) providing a two or three dimensional structure of predetermined shape whereby the structure is formed by nucleic acid molecules, in particular, DNA molecules;
b1) providing nucleic acid sequences complementary to predetermined segments of the structure of a), whereby said free nucleic acid sequences have attached thereto metallic nanoparticles and whereby at least two nucleic acid sequences having attached thereto metallic nanoparticles are provided; or
b2) providing metallic nanoparticles being bound to the two or three dimensional structure of predetermined shape whereby the structure is formed of nucleic acid molecules, in particular, DNA molecules ;
c) providing a compound able to irradiate electromagnetic waves;
d) exciting the compound able to irradiate electromagnetic waves with suitable electromagnetic waves;
e) measuring the irradiated electromagnetic waves of the compound;
whereby said at least two nucleic acid sequences having attached thereto metallic nanoparticles or the metallic nanoparticles bound to the structure are arranged on the structure formed by nucleic acid molecules, in particular, DNA molecules to allow formation of an area of electrical field enhancement due to plasmon resonance after excitation with electromagnetic waves, and the compound able to irradiate electromagnetic waves is positioned in between said area of electrical field enhancement due to plasmon resonance of the at least two metallic nanoparticles.

15. The method according to claim 14 wherein a biochemical or chemical entity, like an enzyme, a binding entity, in particular, a ligand, a receptor, an antibody, is positioned in the area of electrical field enhancement due to plasmon resonance of the metallic nanoparticles, preferably positioned by nucleic acid sequences hybridising to or bound to the predetermined structure formed of nucleic acid molecules, having a two or three dimensional structure of predetermined shape.

16. An array according to any one of claims 1 to 11 for use in a method according to claim 14 or 15.

## Patentansprüche

1. Array umfassend i) eine zwei- oder dreidimensionale Struktur einer vorbestimmten Form, wobei die Struktur ausgewählt ist aus Nukleinsäuremolekülen, insbesondere DNA-Molekülen; ii) a) Nukleinsäuresequenzen mit angehängten Metallnanopartikeln, wobei diese Nukleinsäuresequenzen, die Metallnanopartikel angehängt haben an vorbestimmte Segmente der strukturierten Nukleinsäuremoleküle, insbesondere der DNA-Moleküle, mit zwei- oder dreidimensionaler Struktur vorbestimmter Form, positioniert sind; und/oder b) Metallnanopartikel, die an die vorbestimmte Struktur ausgebildet aus Nukleinsäuremolekülen, insbesondere DNA-Molekülen, mit zwei- oder dreidimensionaler Struktur vorbestimmter Form, gebunden sind; wobei diese Metallnanopartikel so angeordnet sind, dass sie die Ausbildung eines Bereichs der elektrischen Feldverstärkung aufgrund von Plasmonresonanz nach Anregung mit elektromagnetischen Wellen erlaubt; iii) Verbindung, die in der Lage ist elektromagnetische Wellen abzustrahlen, angeordnet zwischen zwei Metallnanopartikeln, die an Nukleinsäuresequenzen angehängt sind und/oder gebunden sind an die vorbestimmte Struktur, wobei die Metallnanopartikel so angeordnet sind, um eine Plasmonresonanz auszubilden und diese Verbindung positioniert ist in diesem Bereich der elektrischen Feldverstärkung aufgrund der Plasmonresonanz.

2. Array nach Anspruch 1, wobei eine biochemische oder chemische Entität, wie ein Enzym, eine Bindungsentität, insbesondere ein Ligand, ein Rezeptor, ein Antikörper, positioniert ist im Bereich der elektrischen Feldverstärkung aufgrund der Plasmonresonsanz der Metallnanopartikel, bevorzugt positioniert ist durch Nukleinsäuresequenzen, die an die zwei- oder dreidimensionale Struktur der vorbestimmten Form, wobei die Struktur ausgebildet ist aus Nukleinsäuremolekülen, hybridisieren oder gebunden sind.

3. Array nach einem der vorherigen Ansprüche, wobei die Struktur ausgebildet aus Nukleinsäuremolekülen, insbesondere DNA-Molekülen, eine Origamistruktur ist, insbesondere eine DNA-Origamistruktur.

4. Array nach einem der vorherigen Ansprüche, wobei die Metallnanopartikel ausgebildet sind aus Plasmonmaterial ausgewählt aus Silber, Aluminium, Gold, Kupfer, Palladium und/oder Platin.

5. Array nach einem der vorherigen Ansprüche, wobei die Metallnanopartikel eine durchschnittliche Größe von 10 nm bis 500 nm, bevorzugt von 10 nm bis 200 nm aufzeigen.

6. Array nach einem der vorherigen Ansprüche, wobei die Beabstandung der mindestens zwei Nanopartikel angehängt an die Nukleinsäuresequenzen und diese Nukleinsäuresequenzen gebunden sind an die Nukleinsäurestruktur, insbesondere die DNA-Struktur, von 2 nm bis 50 nm ist.

7. Array nach einem der vorherigen Ansprüche, wobei die Verbindung, die elektromagnetische Wellen abstrahlen kann, ein Chromophor, insbesondere ein Fluorophor ist.

8. Array nach einem der vorherigen Ansprüche, wobei die Verbindung, die elektromagnetische Wellen abstrahlen kann, nicht kovalent gebunden ist oder direkt hybridisiert ist mit den Metallnanopartikeln.

9. Array nach einem der vorherigen Ansprüche, wobei die vorbestimmte Struktur ausgewählt aus Nukleinsäuremolekülen insbesondere DNA-Molekülen, wie die Origamistruktur, angeheftet ist an eine Oberfläche durch kovalente oder nicht-kovalente Bindung.

10. Array nach einem der vorherigen Ansprüche zur Verwendung in der Einzelmolekülanalyse.

11. Bioassay umfassen einen Array nach einem der vorherigen Ansprüche, wobei dieser Bioassay ein Assay ist, der eine Einzelmolekülanalyse von Biomolekülen erlaubt.

12. Assaysystem umfassend einen Array nach einem der Ansprüche 1 bis 11, eine Quelle mit elektromagnetischen Wellen zur Anregung der Verbindung, die elektromagnetische Wellen abstrahlen kann und, gegebenenfalls, Nachweismittel zum Nachweis der abgestrahlten elektromagnetischen Wellen.

13. Kit zur Messung der Fluoreszenz einzelner Moleküle, das Kit umfasst einen Array nach einem der Ansprüche 1 bis 11.

14. Verfahren zur Messung der Abstrahlung von elektromagnetischen Wellen einer Verbindung, das Verfahren umfasst
a) Bereitstellen einer zwei- oder dreidimensionalen Struktur vorbestimmter Form, wobei die Struktur ausgebildet ist aus Nukleinsäuremolekülen, insbesondere DNA-Molekülen;
b1) Bereitstellen von Nukleinsäuresequenzen komplementär zu vorbestimmten Segmenten der Struktur gemäß a), wobei diese freien Nukleinsäuresequenzen Metallnanopartikel angehängt haben und wobei mindestens zwei Nukleinsäuresequenzen, die Metallnanopartikel angehängt haben, bereitgestellt werden; oder
b2) Bereitstellen von Metallnanopartikeln, die an die zwei- oder dreidimensionale Struktur vorbestimmter Form gebunden sind, wobei die Struktur ausgebildet ist aus Nukleinsäuremolekülen, insbesondere DNA-Molekülen;
c) Bereitstellen einer Verbindung, die elektromagnetische Wellen abstrahlen kann;
d) Anregen der Verbindung, die elektromagnetische Wellen abstrahlen kann mit geeigneten elektromagnetischen Wellen;
e) Messen der abgestrahlten elektromagnetischen Wellen der Verbindung;
wobei diese mindestens zwei Nukleinsäuresequenzen mit angehängten Metallnanopartikeln oder die Metallnanopartikel gebunden an die Struktur so angeordnet sind auf der Struktur ausgebildet durch die Nukleinsäuremoleküle, insbesondere DNA-Moleküle, dass sie die Ausbildung eines Bereichs einer elektronischen Feldverstärkung aufgrund von Plasmonresonanz nach Anregung mit elektromagnetischen Wellen erlaubt, und die Verbindung, die elektromagnetische Wellen abstrahlen kann, angeordnet ist zwischen diesem Bereich der elektrischen Feldverstärkung aufgrund der Plasmonresonanz der mindestens zwei Metallnanopartikel.

15. Verfahren nach Anspruch 14, wobei eine biochemische oder chemische Entität, wie ein Enzym, eine Bindungsentität, insbesondere ein Ligand, ein Rezeptor, ein Antikörper, in den Bereich der elektrischen Feldverstärkung aufgrund der Plasmonresonanz der Metallnanopartikel positioniert ist, bevorzugt positioniert durch Nukleinsäuresequenzhybridisierung oder Bindung an die vorbestimmte Struktur ausgebildet aus Nukleinsäuremolekülen, die eine zwei- oder dreidimensionale Struktur vorbestimmter Form hat.

16. Array nach einem der Ansprüche 1 bis 11 zur Verwendung in einem Verfahren nach Anspruch 14 oder 15.

## Revendications

1. Réseau comprenant i.) une structure bidimensionnelle ou tridimensionnelle de forme prédéterminée, moyennant quoi la structure est formée de molécules d'acide nucléique en particulier, de molécules d'ADN ; ii.) a) des séquences d'acide nucléique auxquelles sont attachées des nanoparticules métalliques, moyennant quoi lesdites séquences d'acide nucléique auxquelles sont attachées des nanoparticules métalliques sont positionnées à des segments prédéterminés des molécules d'acide nucléique structurées en particulier, des molécules d'ADN, ayant une structure bidimensionnelle ou tridimensionnelle de forme prédéterminée ; et/ou b) des nanoparticules métalliques étant liées à la structure prédéterminée formée de molécules d'acide nucléique, en particulier de molécules d'ADN, ayant une structure bidimensionnelle ou tridimensionnelle de forme prédéterminée ; moyennant quoi lesdites nanoparticules métalliques sont agencées pour permettre la formation d'une zone d'accentuation de champ électrique due à une résonance plasmonique après excitation avec des ondes électromagnétiques ; iii.) un composé apte à irradier des ondes électromagnétiques positionné entre deux nanoparticules métalliques qui sont attachées aux séquences d'acide nucléique et/ou liées à la structure prédéterminée, moyennant quoi lesdites nanoparticules métalliques sont agencées pour permettre la formation de résonance plasmonique et ledit composé est positionné dans ladite zone d'accentuation de champ électrique due à une résonance plasmonique.

2. Réseau selon la revendication 1, dans lequel une entité biochimique ou chimique, comme une enzyme, une entité de liaison, en particulier, un ligand, un récepteur, un anticorps, est positionnée dans la zone d'accentuation de champ électrique due à une résonance plasmonique des nanoparticules métalliques, de préférence positionnée par des séquences d'acide nucléique s'hybridant avec ou liées à la structure bidimensionnelle ou tridimensionnelle de forme prédéterminée, moyennant quoi la structure est formée de molécules d'acide nucléique.

3. Réseau selon l'une quelconque des revendications précédentes, dans lequel la structure formée des molécules d'acide nucléique, en particulier des molécules d'ADN, est une structure origami, en particulier une structure ADN-origami.

4. Réseau selon l'une quelconque des revendications précédentes, moyennant quoi les nanoparticules métalliques sont choisies parmi un matériau plasmonique choisi parmi l'argent, l'aluminium, l'or, le cuivre, le palladium et/ou le platine.

5. Réseau selon l'une quelconque des revendications précédentes, moyennant quoi les nanoparticules métalliques ayant une taille moyenne de 10 nm à 500 nm, de préférence de 10 nm à 200 nm.

6. Réseau selon l'une quelconque des revendications précédentes, moyennant quoi l'espacement entre les au moins deux nanoparticules attachées aux séquences d'acide nucléique et lesdites séquences d'acide nucléique sont liées à la structure d'acide nucléique, en particulier la structure d'ADN, est de 2 nm à 50 nm.

7. Réseau selon l'une quelconque des revendications précédentes, moyennant quoi le composé apte à irradier des ondes électromagnétiques est un chromophore, en particulier un fluorophore.

8. Réseau selon l'une quelconque des revendications précédentes, moyennant quoi le composé apte à irradier des ondes électromagnétiques n'est pas lié de manière covalente ou directement hybridé avec les nanoparticules métalliques.

9. Réseau selon l'une quelconque des revendications précédentes, moyennant quoi la structure prédéterminée formée de molécules d'acide nucléique, en particulier les molécules d'ADN, comme la structure origami, est attachée à une surface par liaison covalente ou non covalente.

10. Réseau selon l'une quelconque des revendications précédentes, pour une utilisation dans une analyse de molécule unique.

11. Biodosage comprenant un réseau selon l'une quelconque des revendications précédentes, moyennant quoi ledit biodosage est un dosage permettant une analyse de molécule unique de biomolécules.

12. Système de dosage comprenant un réseau selon l'une quelconque des revendications 1 à 11, une source pour exciter avec des ondes électromagnétiques le composé apte à irradier des ondes électromagnétiques et, facultativement, des moyens de détection pour détecter les ondes électromagnétiques irradiées.

13. Nécessaire de mesure de fluorescence de molécule unique, le nécessaire comprenant un réseau selon l'une quelconque des revendications 1 à 11.

14. Procédé de mesure de l'irradiation d'ondes électromagnétiques d'un composé, le procédé comprenant
a) la fourniture d'une structure bidimensionnelle ou tridimensionnelle de forme prédéterminée, moyennant quoi la structure est formée par des molécules d'acide nucléique, en particulier des molécules d'ADN ;
b1) la fourniture de séquences d'acide nucléique complémentaires à des segments prédéterminés de la structure de a), moyennant quoi lesdites séquences d'acide nucléique libres ont des nanoparticules métalliques attachées et moyennant quoi au moins deux séquences d'acide nucléique auxquelles des nanoparticules métalliques sont attachées sont fournies ; ou
b2) la fourniture de nanoparticules métalliques qui sont liées à la structure bidimensionnelle ou tridimensionnelle de forme prédéterminée, moyennant quoi la structure est formée de molécules d'acide nucléique, en particulier de molécules d'ADN ;
c) la fourniture d'un composé apte à irradier des ondes électromagnétiques ;
d) l'excitation du composé apte à irradier des ondes électromagnétiques avec des ondes électromagnétiques adéquates ;
e) la mesure des ondes électromagnétiques irradiées du composé ;
moyennant quoi lesdites au moins deux séquences d'acide nucléique auxquelles sont attachées des nanoparticules métalliques ou les nanoparticules métalliques liées à la structure sont agencées sur la structure formée par des molécules d'acide nucléique, en particulier des molécules d'ADN pour permettre la formation d'une zone d'accentuation de champ électrique due à une résonance plasmonique après excitation avec des ondes électromagnétiques, et le composé apte à irradier des ondes électromagnétiques est positionné entre ladite zone d'accentuation de champ électrique due à une résonance plasmonique des au moins deux nanoparticules métalliques.

15. Procédé selon la revendication 14, dans lequel une entité biochimique ou chimique, comme une enzyme, une entité de liaison, en particulier un ligand, un récepteur, un anticorps, est positionnée dans la zone d'accentuation de champ électrique due à une résonance plasmonique des nanoparticules métalliques, de préférence positionnée par des séquences d'acide nucléique s'hybridant avec ou liées à la structure prédéterminée formée de molécules d'acide nucléique, ayant une structure bidimensionnelle ou tridimensionnelle de forme prédéterminée.

16. Réseau selon l'une quelconque des revendications 1 à 11, pour une utilisation dans un procédé selon la revendication 14 ou 15.
